# FASCICULE DE BREVET EUROPEEN

(11) **EP 3 389 437 B1**
(45) Date de publication et mention de la délivrance du brevet: **14.08.2019**
(21) Numéro de dépôt: 16808994.4
(22) Date de dépôt: 06.12.2016
(51) Int. Cl.: A45D 34/00

(54) **DISPOSITIF DE CONDITIONNEMENT ET DE DISTRIBUTION D'UN PRODUIT**
VORRICHTUNG ZUM VERPACKEN UND AUSGEBEN EINES PRODUKTS
DEVICE FOR PACKAGING AND DISPENSING A PRODUCT

(30) Priorité: 17.12.2015 FR 1562617
(43) Date de publication de la demande: 24.10.2018
(73) Titulaire: SEB S.A., 69130 Ecully (FR)
(72) Inventeur: PLANARD-LUONG, Thi Hong Lien, 91440 Bures Sur Yvette (FR); MANDICA, Franck, 69340 Francheville (FR); SABATTIER, Johan, 69440 Mornant (FR); FEREYRE, Régis, 69003 Lyon (FR)
(74) Mandataire: Bourrières, Patrice
(86) Numéro de dépôt international: PCT/EP2016/079932
(87) Numéro de publication internationale: WO 2017/102458

(56) Documents cités:
- DE-A1- 2 323 762
- US-A- 4 157 771
- US-A- 5 281 202

## Description

La présente invention concerne les dispositifs de conditionnent et de distribution comportant une recharge contenant un produit à distribuer, et plus particulièrement les dispositifs d'iontophorèse.

Il est connu de conditionner un produit dans une poche souple et de soumettre celle-ci à l'action d'un organe presseur pour forcer le produit à s'écouler à l'extérieur de la poche sous l'effet de la poussée exercée par l'organe presseur.

EP 0 539 667 A1 décrit un dispositif de vidage d'une poche souple comportant un mécanisme à genouillère pour solliciter en déplacement un plateau presseur au contact de la poche. L'intérêt d'un tel mécanisme est d'obtenir une poussée sensiblement constante, quel que soit le degré de vidage de la poche.

US 4 157 771 divulgue un autre exemple de dispositif de vidage, lui aussi à genouillère, avec un plateau presseur guidé en déplacement par quatre guides.

US 1 776 137 décrit un dispositif de vidage agencé pour agir sur un tube de dentifrice; ce dernier est reçu entre deux plateaux presseurs reliés entre eux par un mécanisme à genouillère. A la différence des dispositifs décrits dans les publications EP 0 539 667 A1 et US 4 157 771, aucun ressort de rappel n'est prévu pour solliciter en déplacement les plateaux presseurs, et ceux -ci ne sont sollicités que lorsque l'utilisateur agit dessus.

Il existe un besoin pour perfectionner encore les dispositifs de conditionnement et de distribution afin de mettre le contenu d'une recharge sous pression et utiliser cette pression pour en distribuer le contenu.

Il y a notamment un intérêt pour un dispositif qui soit particulièrement fiable, compact, relativement peu coûteux à réaliser, et qui permette un vidage satisfaisant de la recharge.

L'invention atteint cet objectif grâce à un dispositif de conditionnement et de distribution, de préférence un dispositif de traitement à l'aide d'un courant électrique, comportant :
- une recharge contenant un produit à distribuer, comportant un réservoir défini au moins partiellement par une paroi flexible,
- un organe presseur pour appuyer sur la paroi flexible afin de mettre sous pression le produit contenu à l'intérieur,
caractérisé par le fait que l'organe presseur comporte deux volets articulés entre eux, reliés chacun à un système mécanique sollicitant le volet en appui contre la recharge.

Dans l'invention, le fait d'utiliser au moins deux volets presseurs articulés entre eux permet d'obtenir un bon vidage de la recharge sous un faible encombrement. Les deux volets peuvent être sollicités en pivotement l'un par rapport à l'autre dans le sens d'une fermeture de l'angle existant entre eux, ce qui peut chasser le produit vers l'axe médian de la recharge, et faciliter l'évacuation de son contenu.

De préférence, les volets comportent des reliefs de guidage à leurs extrémités avant et arrière, qui coopèrent avec le reste du dispositif. Ces reliefs de guidage sont de préférence réalisés sous la forme d'ergots venus de moulage de matière thermoplastique avec les volets.

Chaque volet peut comporter un ergot qui coopère avec un relief correspondant durant la descente du volet accompagnant le vidage de la poche. Cette coopération tend à faire pivoter le volet en rapprochement de l'autre volet. L'ergot vient de préférence en appui contre la périphérie extérieure d'une bague qui définit par ailleurs une ouverture à travers laquelle est engagée une canule de sortie du produit de la recharge.

En particulier, chaque volet peut avantageusement comporter un ergot à l'avant et un ergot à l'arrière, lesquels sont guidés dans les fentes correspondantes durant la phase de descente, de façon à assurer un vidage optimal de la recharge.

Lorsque les volets descendent, ils tendent à reculer, c'est-à-dire qu'ils se déplacent en éloignement de l'ouverture ci-dessus ; les ergots, en venant en appui contre la bague, peuvent favoriser une inclinaison des volets vers l'avant et vers le haut, favorable à un vidage du fond de la recharge.

Une fois un recul suffisant atteint, les ergots cessent de venir au contact de la bague, et les volets ne sont pas contraints par celle-ci dans leur orientation.

Le système mécanique comporte de préférence, pour chaque volet, un coulisseau sollicité en déplacement selon un axe par un ressort et une biellette reliée de façon articulée à ses extrémités respectivement au coulisseau et au volet, de telle sorte qu'un déplacement du coulisseau selon ledit axe, tendant à augmenter l'angle de la biellette avec celui-ci, s'accompagne d'une poussée exercée par la biellette sur le volet.

Les deux coulisseaux peuvent être reliés entre eux par un axe commun servant d'articulation aux deux biellettes. Cet axe peut être encliqueté à chacune de ses extrémités dans un logement du coulisseau ouvert dans une direction opposée au ressort. Les ressorts sont de préférence hélicoïdaux et engagés sur des tiges sur lesquelles se déplacent les coulisseaux.

La recharge peut être allongée selon un axe longitudinal et les volets sont de préférence articulés entre eux autour d'un axe géométrique d'articulation sensiblement parallèle à l'axe longitudinal de la recharge. En particulier, les deux volets peuvent être chacun reliés de façon articulée à un élément d'appui pivotant, pouvant accompagner en pivotant le mouvement des volets. Cet élément d'appui pivotant complète avec la biellette la genouillère et il est avantageusement formé par une branche pourvue en extrémité d'un crochet servant d'axe pour relier la biellette au volet associé. Les branches peuvent former partie d'une boucle ouverte dont le côté opposé aux branches vient en appui dans le fond d'un crochet fixe du dispositif. De préférence, la boucle est réalisée en fil à ressort. Ainsi, la déformation élastique de la boucle autorise un léger pivotement des volets l'un par rapport à l'autre, tout en sollicitant ces derniers dans une position de repos prédéfinie
La recharge comporte de préférence une poche souple définissant la paroi flexible précitée. La recharge peut comporter à l'avant une canule de distribution, comme mentionné plus haut.

Le dispositif comporte avantageusement un boîtier présentant un logement pour recevoir la recharge, et une tête de traitement par laquelle le produit est distribué. De préférence, la tête de traitement est fixée de façon amovible sur le boîtier, et peut être retirée pour la mise en place et l'enlèvement de la recharge.

La recharge peut contenir un produit cosmétique ou dermatologique, de préférence conducteur électrique.

La distribution du produit peut s'effectuer en ouvrant sélectivement un passage permettant l'établissement d'une communication fluidique entre l'intérieur du réservoir et au moins un orifice de sortie du dispositif. De préférence, le dispositif comporte une électrovanne qui définit ce passage, ou une vanne actionnable manuellement pour distribuer le produit.

L'invention pourra être mieux comprise à la lecture de la description détaillée qui va suivre, d'un exemple de mise en oeuvre non limitatif de celle-ci, et à l'examen du dessin annexé, sur lequel :
- la figure 1 représente de façon schématique, en perspective, un dispositif de conditionnement et de distribution selon l'invention, la recharge étant représentée isolément à l'extérieur de l'appareil,
- la figure 2 est une vue en perspective, selon un autre angle de vue, de l'appareil de la figure 1, l'organe presseur occupant sur les figures 1 et 2 une position différente,
- la figure 3 est une vue de l'appareil après enlèvement du capot de protection de la tête de traitement,
- la figure 4 représente isolément les volets et le système mécanique sollicitant ceux-ci en appui contre la recharge, dans une position basse correspondant au vidage de la recharge,
- la figure 5 est une vue analogue à la figure 4, dans une position haute, la recharge étant pleine,
- les figures 6 et 7 sont des vues en perspective des volets et du système mécanique,
- la figure 8 représente partiellement le système mécanique,
- la figure 9 est une coupe transversale du dispositif,
- les figures 10 et 11 sont deux vues en perspective illustrant le déplacement des volets relativement à la tête de l'appareil au cours du vidage de la recharge, et
- la figure 12 illustre le guidage des volets à l'arrière.

Le dispositif 1 de conditionnement et de distribution selon l'invention comporte un appareil 10 qui reçoit une recharge 20 contenant un produit à appliquer sur la région à traiter à l'aide de l'appareil.

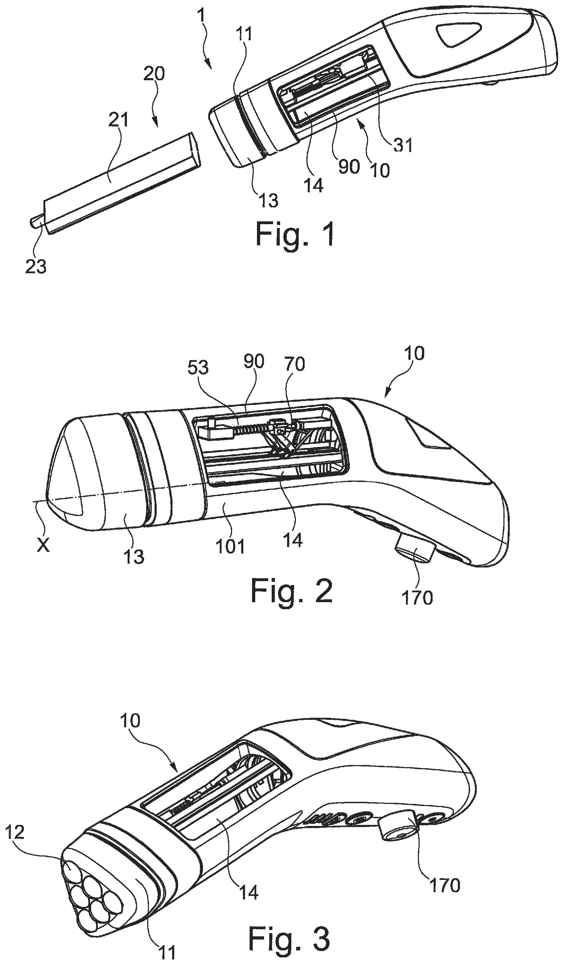

Dans l'exemple considéré, le produit contenu dans la recharge 20 est un produit cosmétique ou dermatologique et l'appareil 10 est un appareil d'iontophorèse destiné au traitement des matières kératiniques humaines à l'aide d'un courant électrique. L'appareil 10 comporte à cet effet, comme visible sur la figure 3 notamment, une tête de traitement 11 comportant en l'espèce une pluralité de billes applicatrices 12 qui permettent d'appliquer le produit sur la région à traiter tout en déplaçant l'appareil à son contact. L'appareil 10 peut comporter un circuit électronique (non apparent) permettant de soumettre la région à traiter à un courant électrique simultanément à l'application du produit contenu dans la recharge 20. L'appareil peut comporter une électrode au contact de la composition et une contre-électrode tenue par l'utilisateur. La composition est conductrice de l'électricité.

L'appareil 10 peut comporter, comme illustré, un bouton 170 de réglage de l'intensité du courant et d'autres boutons et/ou indicateurs utiles au contrôle de l'appareil.

En l'absence d'utilisation, la tête de traitement 11 peut être recouverte par un capot amovible 13, comme illustré sur les figures 1 et 2.

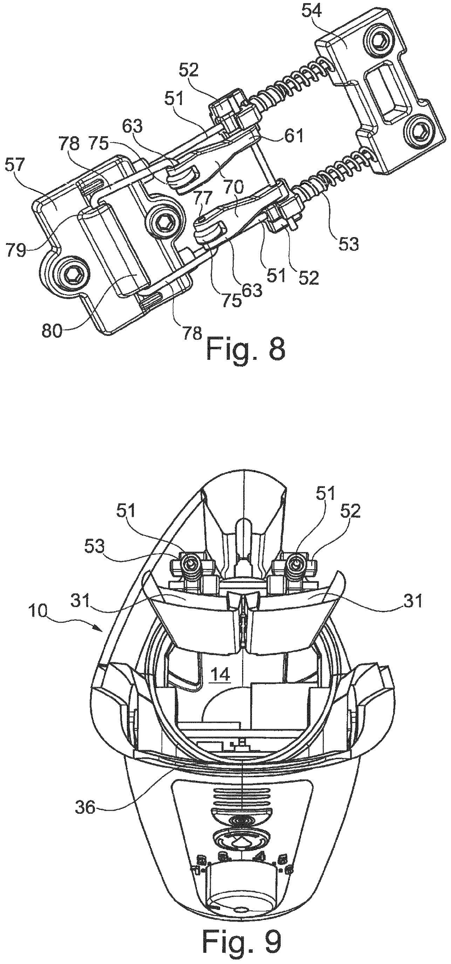

La recharge 20 est reçue à l'intérieur de l'appareil 10 dans un logement 14 visible sur la figure 9 notamment, la recharge 20 n'étant pas représentée sur les figures à l'intérieur de l'appareil 10 dans un souci de clarté. Le boîtier de l'appareil présente une fenêtre 90 qui permet d'accéder au logement 14.

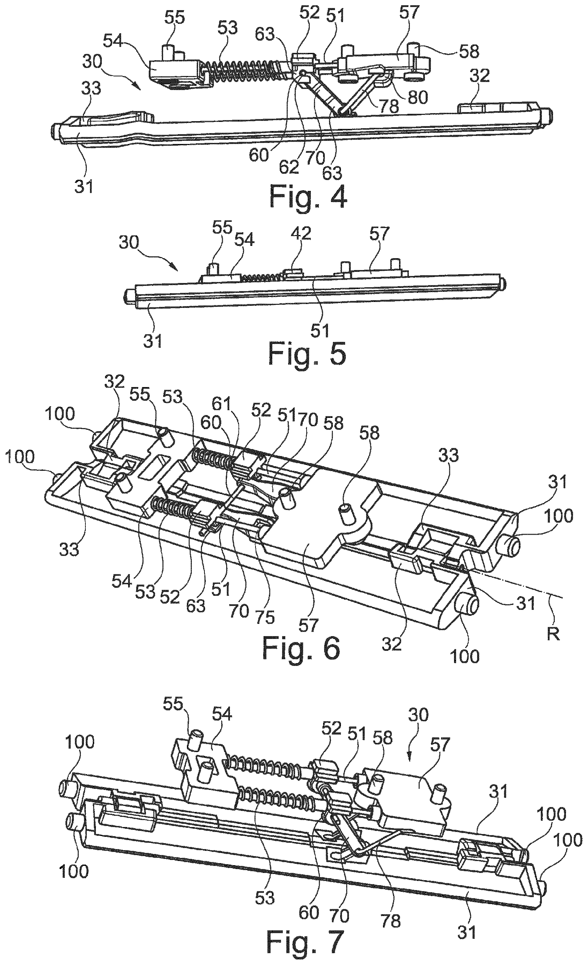

L'appareil 10 comporte un dispositif 30 de vidage de la recharge 20 qui comprend, comme on peut le voir notamment sur les figures 4 à 7 deux volets 31, constituant ensemble un organe presseur venant au contact de la face supérieure 21 de la recharge 20 lorsque cette dernière est en place dans l'appareil 10. Les deux volets 31 sont reliés ensemble de façon articulée autour d'un axe géométrique d'articulation R qui est sensiblement parallèle à l'axe longitudinal X du logement recevant la recharge 20. Cette dernière est elle-même de forme allongée autour d'un axe longitudinal qui coïncide sensiblement avec l'axe longitudinal X lorsque la recharge 20 est en place dans l'appareil 10.

Dans l'exemple considéré, les volets 31 présentent des éléments de charnière mâles et femelles 32 et 33 qui permettent de réaliser une telle articulation, les éléments d'articulation 32 et 33 étant prévus respectivement à l'avant et à l'arrière des volets 31, c'est-à-dire près de leurs extrémités longitudinales.

Des ergots 100 sont présents à l'extrémité des volets 31. Leur rôle est précisé plus loin.

Pour solliciter l'organe presseur constitué par les volets 31 en déplacement vers le bas, c'est-à-dire vers la paroi 36 constituant le fond du logement 14 recevant la recharge 20, un système mécanique est prévu. Ce système mécanique comporte deux tiges 51, sensiblement parallèles à l'axe longitudinal X, sur lesquelles se déplacent des coulisseaux 52, chacun sous l'effet de rappel d'un ressort hélicoïdal 53 disposé sur la tige 51 correspondante. L'extrémité de chaque ressort opposée au coulisseau 52 vient en appui sur un élément de butée 54 qui sert également d'entretoise pour les tiges 51. Cet élément de butée 54 présente des fûts 55 de réception des vis, qui permettent sa fixation sur le corps 101 du boîtier de l'appareil 10. Les extrémités des tiges 51 opposées à l'élément de butée 54 sont maintenues par une pièce 57 qui est également fixée sur le corps du boîtier par des fûts 58, dans lesquels sont engagées des vis non apparentes.

L'élément de butée 54 et la pièce 57 sont fixes l'un relativement à l'autre à l'intérieur du boîtier 101 lors du fonctionnement du dispositif 30.

Les coulisseaux 52 sont reliés entre eux par un axe 60 qui est orienté perpendiculairement à celui des tiges 51 et qui est formé dans l'exemple considéré par une tige métallique. Cette dernière est encliquetée dans des logements 62 formés dans des ailes 63 réalisées par moulage de matière avec les coulisseaux 52. Les ailes 63 prolongent les coulisseaux 52 sous les tiges 51.

Une biellette 70 relie chaque coulisseau 52 à un volet correspondant 31. Ces biellettes 70 comportent une extrémité 61 qui est traversée par l'axe 60 et une extrémité opposée 63 qui est reliée de façon articulée au volet 31. L'extrémité 63 forme, comme on le voit sur la figure 8 notamment, une chape entre les branches de laquelle est engagé un élément d'articulation 75 réalisé d'une seule pièce par moulage avec le volet correspondant 31. L'extrémité 63 de chaque biellette 70 et l'élément d'articulation 75 associé sont traversés par un crochet 77 formé en coudant une branche 78 d'une boucle en forme de cadre ouvert. Le côté 79 de la boucle opposé à l'ouverture est reçu dans un crochet 80 venu de moulage avec la pièce 57, sur sa face tournée vers les volets 31. La boucle est formée en fil à ressort, ce qui permet aux branches 78 de fléchir légèrement l'une relativement à l'autre et de pouvoir effectuer également une rotation par torsion du côté de la boucle reçu dans le crochet 80.

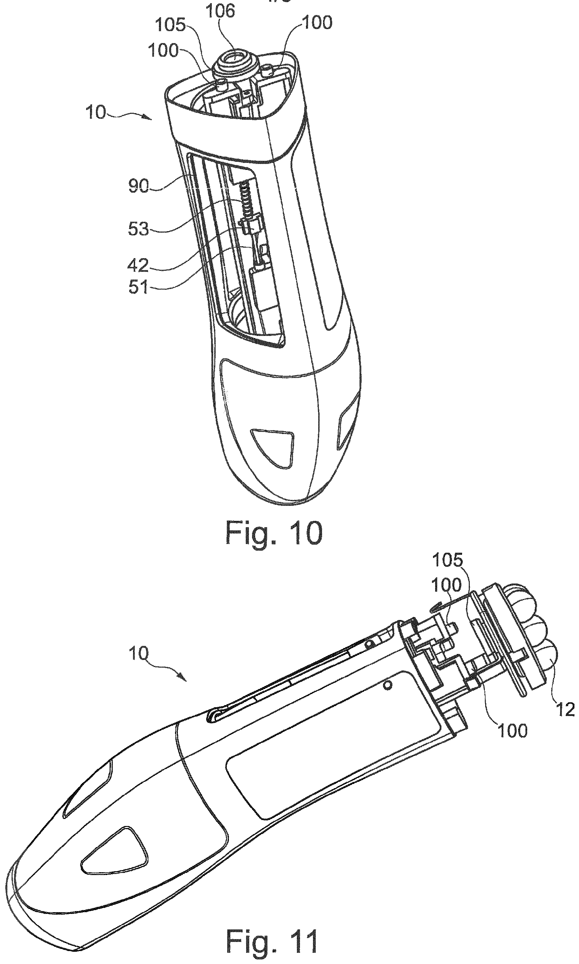

Si l'on se réfère aux figures 10 et 11, on voit que la tête 11 comporte une bague 105 définissant une ouverture 106 d'introduction de la canule de distribution 23 présente à l'avant de la recharge 20.

La tête 11 présente des canaux qui guident le produit vers les billes 12 et permet au produit de quitter le dispositif par le jeu existant entre les billes 12 et leurs logements.

Lorsque la recharge 20 est pleine, les volets 31 sont en position haute, comme illustré à la figure 10. Les ressorts 53 sont comprimés au maximum, et les volets 31 sont positionnés le plus en avant vers la tête 11.

Les ergots 100 présents à l'extrémité avant se positionnent au-dessus de la bague 105.

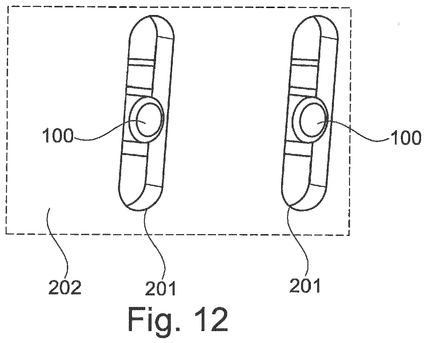

Les ergots 100 situés à l'arrière sont engagés dans des fentes respectives 201 d'une paroi 202 du dispositif 1, comme illustré à la figure 12. Les fentes 201 sont parallèles entre elles et les ergots 100 descendent dans celles-ci lors du vidage de la recharge 20.

De préférence, le jeu de guidage des ergots 100 est assez important, pour leur permettre de pivoter facilement.

Le fonctionnement du dispositif 1 est le suivant.

En vue d'introduire la recharge 20 dans l'appareil 10, l'utilisateur commence par enlever la tête de traitement 11 afin d'accéder par l'avant au logement 14. L'utilisateur fixe la recharge 20 sur la tête 11 en insérant la canule 23 dans l'ouverture 106 de la tête de traitement, puis introduit la recharge 20 ainsi couplée à la tête de traitement 11 dans le logement 14, en utilisant éventuellement la fenêtre 90 ménagée dans le corps du boîtier pour amener au préalable les volets 31 en position haute.

Une fois la recharge 20 en place, les volets 31 s'appliquent contre celle-ci et la face inférieure de la recharge est en appui contre la paroi 36 définissant le fond du logement 14, à l'opposé des volets 31.

Les biellettes 70 agissent à la manière d'une genouillère, ce qui permet de maintenir une force d'appui des volets 31 sur la recharge 20 sensiblement constante au cours de l'élongation des ressorts 53. Ainsi, en sortie de la recharge 20, le produit peut être distribué avec un débit sensiblement constant, lorsque la communication mettant la canule de distribution 53 en liaison avec le ou les orifices de distribution est ouverte.

Un système de vanne ou d'électrovanne non représenté permet d'ouvrir cette communication fluidique entre l'intérieur de la recharge et un ou plusieurs orifices de distribution, soit en réponse à l'actionnement d'un bouton de distribution par l'utilisateur, soit de façon automatique lorsque l'appareil détermine qu'une telle distribution doit être déclenchée.

Le système de vanne comporte par exemple un système d'ouverture/fermeture dans lequel le produit circule dans un conduit souple et un mécanisme venant écraser le conduit pour le fermer, et qui s'en écarte pour rétablir le passage du produit.

Lors de l'introduction de la recharge, le système de distribution est fermé, de sorte qu'on évite une sortie accidentelle de produit même si l'utilisateur appuie sur la recharge avec des doigts.

L'appareil comprend un obturateur soit lié à la canule de sortie 23 de la recharge 20 soit lié à l'embout qui s'ouvre à la commande de l'utilisateur. Par exemple, l'obturateur comporte un volet de mise en pression grâce à un ressort. Le volet peut être mis en retrait par appui d'un bouton s'opposant au ressort permettant le recul du volet ce qui permet de libérer le passage de la formule. Le volume de la recharge 20 est par exemple compris entre 10 et 20 ml, l'invention n'étant toutefois pas limitée à une telle plage de volume.

L'épaisseur de la recharge 20, qui doit être comprimée par les volets 31, est par exemple comprise entre 9 et 13 mm, et la course des volets 31 lors de leur mouvement de descente, en l'absence de recharge 20, est de préférence supérieure.

## Revendications

1. Dispositif de conditionnement et de distribution, de préférence dispositif de traitement à l'aide d'un courant électrique, comportant :
- Une recharge (20) contenant un produit à distribuer, comportant un réservoir défini au moins partiellement par une paroi flexible,
- un organe presseur (31) pour appuyer sur la paroi flexible afin de mettre sous pression le produit contenu à l'intérieur,
**caractérisé par le fait que** l'organe presseur comporte deux volets (31) articulés entre eux, reliés chacun à un système mécanique (30) sollicitant le volet en appui contre la recharge.

2. Dispositif selon la revendication 1, le système mécanique comportant pour chaque volet un coulisseau (42) sollicité en déplacement selon un axe par un ressort (53) et une biellette (70) reliée de façon articulée à ses extrémités respectivement au coulisseau et au volet, de telle sorte qu'un déplacement du coulisseau selon son axe tendant à augmenter l'angle de la biellette avec ledit axe s'accompagne d'une poussée exercée par la biellette sur le volet.

3. Dispositif selon la revendication 2, les deux coulisseaux étant reliés entre eux par un axe commun (60) servant d'articulation aux deux biellettes.

4. Dispositif selon la revendication précédente, l'axe (60) étant encliqueté dans un logement du coulisseau ouvert dans une direction opposée au ressort.

5. Dispositif selon l'une quelconque des revendications 2 à 4, les ressorts (53) étant hélicoïdaux et engagés sur des tiges (51) sur lesquelles se déplacent les coulisseaux.

6. Dispositif selon l'une quelconque des revendications précédentes, la recharge étant allongée selon un axe longitudinal et les volets étant articulés entre eux autour d'un axe d'articulation (R) parallèle à l'axe longitudinal de la recharge.

7. Dispositif selon l'une quelconque des revendications précédentes, les deux volets (31) étant chacun relié de façon articulée à un élément d'appui pivotant (78) pouvant accompagner en pivotant le mouvement des volets.

8. Dispositif selon la revendication 7, l'élément d'appui pivotant étant formé par une branche (78) pourvue en extrémité d'un crochet (77) servant d'axe pour relier la biellette (70) au volet (31).

9. Dispositif selon la revendication 8, les branches (78) formant partie d'une boucle ouverte dont le côté opposé aux branches vient en appui dans le fond d'un crochet fixe (80) du dispositif.

10. Dispositif selon l'une quelconque des revendications précédentes, la boucle étant réalisée en fil à ressort.

11. Dispositif selon l'une quelconque des revendications précédentes, la recharge comportant une poche souple définissant la paroi flexible.

12. Dispositif selon l'une quelconque des revendications précédentes, la recharge comportant à l'avant une canule de distribution (23).

13. Dispositif selon l'une quelconque des revendications précédentes, comportant un boîtier présentant un logement (14) pour recevoir la recharge (20), et une tête de traitement (11) par laquelle le produit est distribué.

14. Dispositif selon la revendication 13, la tête de traitement étant fixée de façon amovible sur le boîtier, et pouvant être retirée pour la mise en place et l'enlèvement de la recharge.

15. Dispositif selon l'une quelconque des revendications 1 à 14, les volets ayant à leurs extrémités avant et arrière des reliefs de guidage (100) coopérant avec le reste du dispositif, ces reliefs étant de préférence réalisés sous la forme d'ergots venus de moulage de matière thermoplastique avec les volets, les ergots arrière étant de préférence guidés dans des fentes correspondantes (201).

16. Dispositif selon l'une quelconque des revendications précédentes, la recharge contenant un produit cosmétique ou dermatologique.

## Patentansprüche

1. Vorrichtung zur Verpackung und Ausgabe, vorzugsweise Vorrichtung zur Behandlung mit einem elektrischen Strom, umfassend:
- Eine Nachfüllpackung (20), die ein auszugebendes Produkt enthält, die einen Behälter umfasst, der zumindest teilweise durch eine flexible Wand gebildet wird,
- ein Druckelement (31) zum Drücken gegen die flexible Wand, um das im Inneren enthaltene Produkt unter Druck zu setzen,
**gekennzeichnet durch** die Tatsache, dass das Druckelement zwei gelenkig miteinander verbundene Klappen (31) umfasst, die jeweils mit einem mechanischen System (30) verbunden sind, welches die Klappe gegen die Nachfüllpackung drückt.

2. Vorrichtung nach Anspruch 1, wobei das mechanische System für jede Klappe ein Gleitstück (42) umfasst, das durch eine Feder (53) und ein Gestänge (70), welches an seinen Enden jeweils mit dem Gleitstück und der Klappe gelenkig verbunden ist, entlang einer Achse in Bewegung versetzt wird, so dass eine Verschiebung des Gleitstücks entlang seiner Achse, die dazu neigt, den Winkel zwischen Gestänge und Achse zu vergrößern, von einem Schub begleitet wird, der von dem Gestänge auf die Klappe ausgeübt wird.

3. Vorrichtung nach Anspruch 2, wobei die beiden Gleitstücke durch eine gemeinsame Achse (60) miteinander verbunden sind, die den beiden Gestängen als Gelenk dient.

4. Vorrichtung nach dem vorstehenden Anspruch, wobei die Achse (60) in eine Aufnahme des Gleitstücks eingerastet ist, das in einer der Feder entgegengesetzten Richtung geöffnet ist.

5. Vorrichtung nach einem der Ansprüche 2 bis 4, wobei die Federn (53) schraubenförmig sind und auf Stangen (51) aufgesteckt sind, auf denen sich die Gleitstücke bewegen.

6. Vorrichtung nach einem der vorstehenden Ansprüche, wobei sich die Nachfüllpackung entlang einer Längsachse erstreckt und die Klappen um eine Gelenkachse (R) parallel zur Längsachse der Nachfüllpackung gelenkig miteinander verbunden sind.

7. Vorrichtung nach einem der vorstehenden Ansprüche, wobei die beiden Klappen (31) jeweils gelenkig mit einem schwenkbaren Auflageelement (78) verbunden sind, das die Bewegung der Klappen durch Schwenken begleiten kann.

8. Vorrichtung nach Anspruch 7, wobei das schwenkbare Auflageelement aus einem Arm (78) gebildet wird, der am Ende mit einem Haken (77) versehen ist, welcher als Achse zum Verbinden des Gestänges (70) mit der Klappe (31) dient.

9. Vorrichtung nach Anspruch 8, wobei die Arme (78) Teil einer offenen Schleife sind, deren den Armen gegenüberliegende Seite gegen die Unterseite eines festen Hakens (80) der Vorrichtung drückt.

10. Vorrichtung nach einem der vorstehenden Ansprüche, wobei die Schleife aus Federdraht besteht.

11. Vorrichtung nach einem der vorstehenden Ansprüche, wobei die Nachfüllpackung eine elastische Tasche umfasst, die die flexible Wand bildet.

12. Vorrichtung nach einem der vorstehenden Ansprüche, wobei die Nachfüllpackung an der Vorderseite eine Ausgabekanüle (23) umfasst.

13. Vorrichtung nach einem der vorstehenden Ansprüche, umfassend ein Gehäuse mit einer Halterung (14) zur Aufnahme der Nachfüllpackung (20) und einem Behandlungskopf (11), durch den das Produkt ausgegeben wird.

14. Vorrichtung nach Anspruch 13, wobei der Behandlungskopf lösbar am Gehäuse befestigt und zum Einsetzen und Entfernen der Nachfüllpackung abnehmbar ist.

15. Vorrichtung nach einem der Ansprüche 1 bis 14, wobei die Klappen an ihrem vorderen und hinteren Ende Führungsreliefs (100) aufweisen, die mit dem Rest der Vorrichtung zusammenwirken, wobei diese Reliefs vorzugsweise in Form von Stiften aus thermoplastischem Kunststoff mit den Klappen geformt sind und wobei die hinteren Stifte vorzugsweise in entsprechenden Schlitzen (201) geführt werden.

16. Vorrichtung nach einem der vorstehenden Ansprüche, wobei die Nachfüllpackung ein kosmetisches oder dermatologisches Produkt enthält.

## Claims

1. Device for packaging and dispensing, preferably treatment device using an electric current, comprising:
- a refill (20) comprising a product to be dispensed, comprising a vessel defined at least partially by a flexible wall,
- a pressing member (31) to press on the flexible wall in order to pressurise the product contained therein,
**characterised in that** the pressing member comprises two flaps (31) hinging with one another, each connected to a mechanical system (30) causing the flap to bear against the refill.

2. Device according to claim 1, the mechanical system comprising for each flap a slide (42) moved along an axis by a spring (53) and a rod (70) connected in a hinged manner at its ends respectively to the slide and to the flap, so that a motion of the slide along its axis tending to increase the angle between the rod and said axis is accompanied by a thrust exerted by the rod on the flap.

3. Device according to claim 2, the two slides being connected to one another by a common axis (60) serving as a hinge for the two rods.

4. Device according to the preceding claim, the axis (60) being snap-fitted in a housing of the slide opening in an opposite direction to the spring.

5. Device according to any one of claims 2 to 4, the springs (53) being coil springs and engaged on stems (51) on which the slides move.

6. Device according to any one of the preceding claims, the refill being elongated along a longitudinal axis and the flaps being hinged with respect to one another about a hinge axis (R) parallel to the longitudinal axis of the refill.

7. Device according to any one of the preceding claims, the two flaps (31) being each connected in a hinged manner to a pivoting support element (78) being able to accompany by pivoting the motion of the flaps.

8. Device according to claim 7, the pivoting support element being formed by a branch (78) provided at its end with a hook (77) serving as an axis to connect the rod (70) to the flap (31).

9. Device according to claim 8, the branches (78) forming a part of an open loop of which the side opposite the branches comes to bear against the bottom of a fixed hook (80) of the device.

10. Device according to any one of the preceding claims, the loop being made of spring wire.

11. Device according to any one of the preceding claims, the refill comprising a flexible pouch defining a flexible wall.

12. Device according to any one of the preceding claims, the refill comprising on the front a dispensing needle (23).

13. Device according to any one of the preceding claims, comprising a casing with a housing (14) to hold the refill (20), and a treatment head (11) through which the product is dispensed.

14. Device according to claim 13, the treatment head being secured in a removable manner to the casing, and being removable for the installation and removal of the refill.

15. Device according to any one of claims 1 to 14, the flaps having at their front and rear ends guiding extrusions (100) cooperating with the rest of the device, said extrusions being preferably made in the form of lugs integrally moulded from a thermoplastic material with the flaps, the rear lugs being preferably guided in corresponding slots (201).

16. Device according to any one of the preceding claims, the refill containing a cosmetic or dermatological product.
